# EUROPEAN PATENT APPLICATION

(11) **EP 4 599 836 A1**
(43) Date of publication of application: **13.08.2025**
(21) Application number: 24382126.1
(22) Date of filing: 09.02.2024
(51) Int. Cl.: A61K 35/74, A61K 39/00, A61P 31/04

(54) **MYCOBACTERIUM BRUMAE FOR PREVENTING AND/OR TREATING INFECTIONS**

(71) Applicant: Fundació Institut de Bioenginyeria de Catalunya (IBEC), 08028 Barcelona (ES); Universitat de Barcelona, 08028 Barcelona (ES); Universitat Autònoma de Barcelona, 08193 Bellaterra (Cerdanyola del Vallès) (ES)
(72) Inventor: TORRENTS SERRA, Eduard, 08911 BADALONA (ES); JULIÁN GÓMEZ, Esther, 08911 BADALONA (ES); CAMPO PÉREZ, Víctor, 08290 CERDANYOLA DEL VALLÈS (ES)
(74) Representative: ZBM Patents - Zea, Barlocci & Markvardsen

(57) **Abstract**

The present disclosure is directed to *Mycobacterium brumae,* a cell lysate, a cell-free extract, a component, or a culture supernatant thereof, as well as compositions thereof, for use in the prevention and/or treatment of an infection or of a disease caused by an infection.

## Description

### Technical Field

The present disclosure pertains to the field of medicine and, in particular, to methods for preventing and/or treating infections.

### Background Art

The misuse of antibiotics has posed unprecedented challenges to human society due to the rapid rise of antimicrobial resistance. Antimicrobial resistance occurs naturally when microbes are exposed to antimicrobial medications. The increasing selection for antimicrobial resistance is not solely caused by the excessive use of antibiotics in healthcare, agriculture, and the environment, but also by inappropriate antibiotic usage. This includes making improper choices, administering inadequate doses, and not adhering to treatment guidelines. Additionally, the treatment of multidrug-resistant bacterial infections that are difficult to address with antibiotics faces limitations. Key factors contributing to these limitations encompass a lack of comprehensive understanding of resistance mechanisms, diminished economic incentives for new drug development, and regulatory hurdles.

ESKAPE pathogens, including *Enterococcus faecium, Staphylococcus aureus, Klebsiella pneumoniae, Acinetobacter baumannii, Pseudomonas aeruginosa,* and *Enterobacter species,* represent some of the most prevalent opportunistic pathogens in hospital-acquired infections. The ESKAPE group of bacteria has an outstanding ability to evade destruction by antibiotics and resist conventional treatment methods, resulting in significant morbidity, mortality among patients, and heightened healthcare resource utilization. Infections associated with ESKAPE pathogens have become a major challenge in determining effective therapeutic approaches, posing a high risk of mortality and increased economic burdens.

In addition to the antibiotic-resistance problematic, virus infections, for which antibiotics are ineffective, are also of great concern. Moreover, emerging infectious diseases are an increasing reality, as sadly revealed by the recent Covid-19 pandemic.

The loss of effectiveness of existing antibiotics and antimicrobials against known pathogens, lack of effective anti-viral agents, and concern about new emerging infectious diseases direly require development of new strategies to combat infections.

### Summary of Invention

The inventors have surprisingly found that administering *Mycobacterium brumae* prevents infections caused by highly virulent and antibiotic-resistant pathogens. As shown in the examples below, when administered to a subject, this particularly *Mycobacterium* is able to prevent and/or treat infections caused by highly virulent pathogens.

Thus, a first aspect of the invention refers to *Mycobacterium brumae* for use in the prevention and/or treatment of an infection. This aspect can be reworded as use of *Mycobacterium brumae* for the manufacture of a medicament for the prevention and/or treatment of an infection. It is also disclosed a method for preventing and/or treating an infection, the method comprising administering *Mycobacterium brumae* to a subject in need thereof.

The examples below effectively show that *Mycobacterium brumae* prevents infections of multiresistant ESKAPE pathogens in an animal model. As shown in the examples, animals primed with *M. brumae* showed complete protection against lethal infections from various nosocomial pathogens such as *Pseudomonas aeruginosa, Klebsiella pneumoniae, Acinetobacter baumannii,* and uropathogenic *Escherichia coli* (UPEC). As a result, disease was effectively prevented. It is highly surprising that *Mycobacterium brumae* can prevent the infection by itself, i.e. without administering any other active ingredient. This potent protective effect of *M. brumae* against highly virulent pathogens could not be expected in view of the prior art. Other mycobacteria species, such as *Mycobacterium bovis bacillus* Calmette-Guérin (BCG), are known as adjuvants in vaccines for preventing infections. However, *M. bovis* BCG is not able to prevent infections caused by the above-mentioned pathogens, as is also shown in the examples below.

Another important advantage of the use of *M. brumae* to prevent or treat infections is that *M. brumae* is non-pathogenic and non-toxic. *Mycobacterium brumae* is defined as biosafety level 1 in all international culture collections (LDG Standards-ATCC), and thus belongs to a group of non-pathogenic environmental mycobacteria. No infections caused by *M. brumae,* either in humans, animals or plants, have been reported. The safety profile of *M. brumae,* together with the surprising effect that has been found for this microorganism in protecting against infections from dangerous pathogens, renders *M. brumae* a convenient new strategy to combat infections without un-desired side effects. Consequently, *Mycobacterium brumae* can be used both in non-viable and viable (live) form, in contrast to other mycobacteria such as *M. bovis* BCG.

A second aspect of the present disclosure refers to *Mycobacterium brumae* for use in the prevention and/or treatment of a disease caused by a pathogen. This aspect can be reworded as use of *Mycobacterium brumae* for the manufacture of a medicament for the prevention and/or treatment of a disease caused by a pathogen. It is also disclosed a method for preventing and/or treating a disease caused by a pathogen, the method comprising administering *Mycobacterium brumae* to a subject in need thereof.

A third aspect of the invention refers to a composition comprising a preventively or therapeutically effective amount of *Mycobacterium brumae* for use in the prevention and/or treatment of an infection or for the prevention and/or treatment of disease caused by a pathogen. This aspect can be reworded as use of a composition comprising a preventively or therapeutically effective amount of *Mycobacterium brumae* together with excipients and/or carriers for the manufacture of a medicament for the prevention and/or treatment of an infection or for the prevention and/or treatment of disease caused by a pathogen. It is also disclosed a method for preventing and/or treating an infection or for preventing and/or treating a disease caused by a pathogen, the method comprising administering a composition comprising a preventively or therapeutically effective amount of *Mycobacterium brumae* to a subject in need thereof.

### Brief Description of Drawings

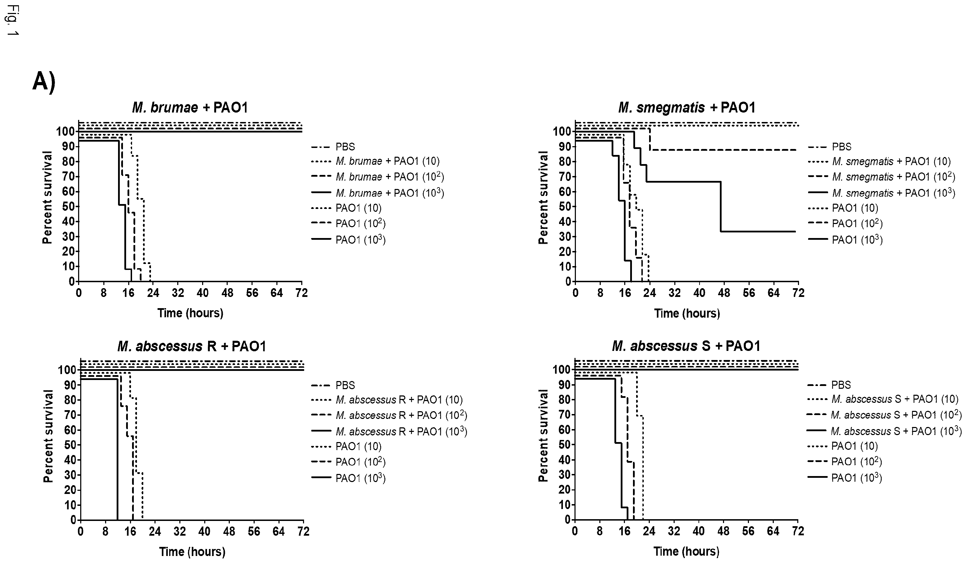

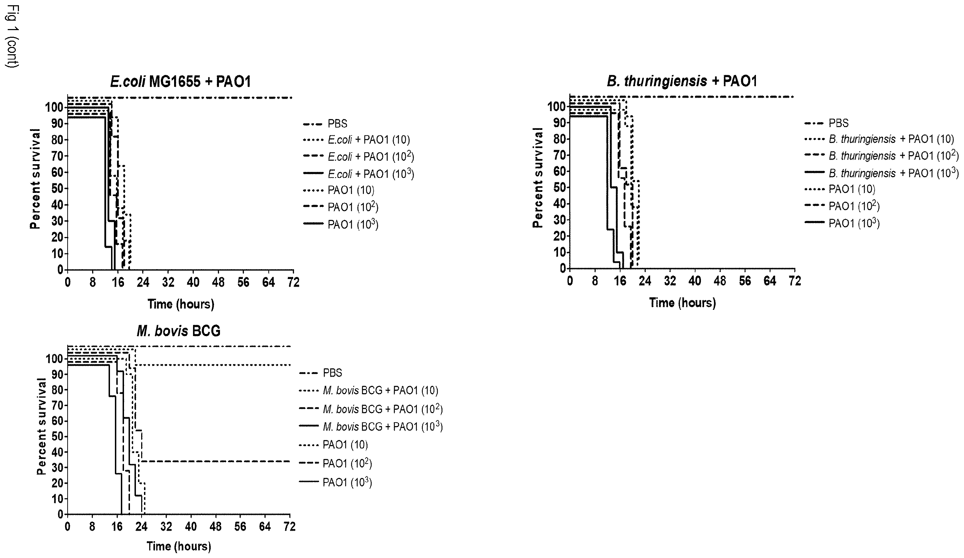

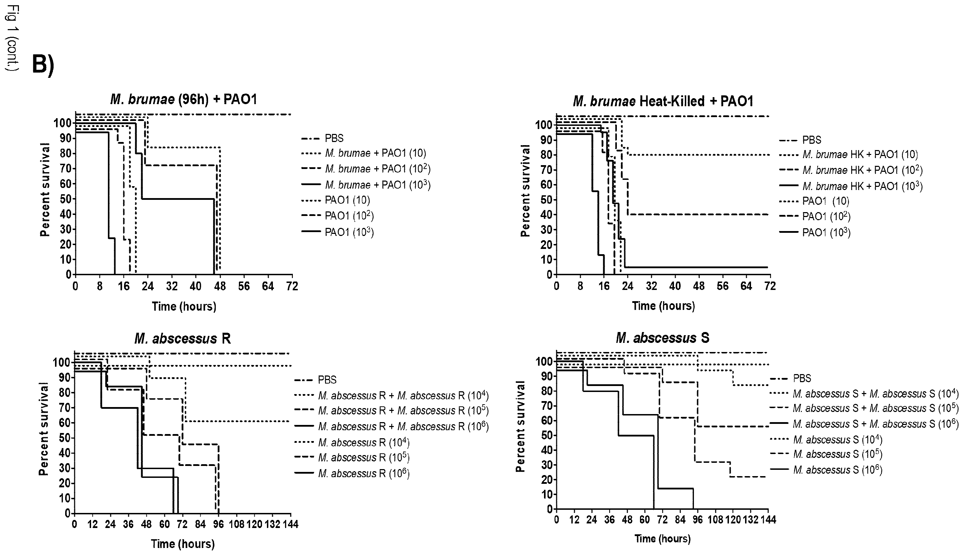

**Figure 1****: Kaplan Meier survival curves of *Galleria mellonella* larvae priming experiments. A)** Priming survival assays treating the larvae (n=30), 24 h before lethal *P. aeruginosa* PAO1 doses, with the mycobacteria (*M. brumae* and *M. smegmatis* (also non-pathogenic), *M. abscessus* Rough (R), Smooth (S) (pathogenic) and *M. bovis* BCG (attenuated)), and *E. coli* MG1655 and *Bacillus thuringiensis* as Gram-negative and Gram-positive controls respectively. **B)** Survival curve of *P. aeruginosa* PAO1-infected larvae 96 h post *M. brumae* priming injection and 24 h post-injection with heat-killed *M. brumae.* Survival curves of larvae primed with *M. abscessus* R and S (pathogens) safe doses and infected 24 h later with lethal doses of the identical mycobacteria.

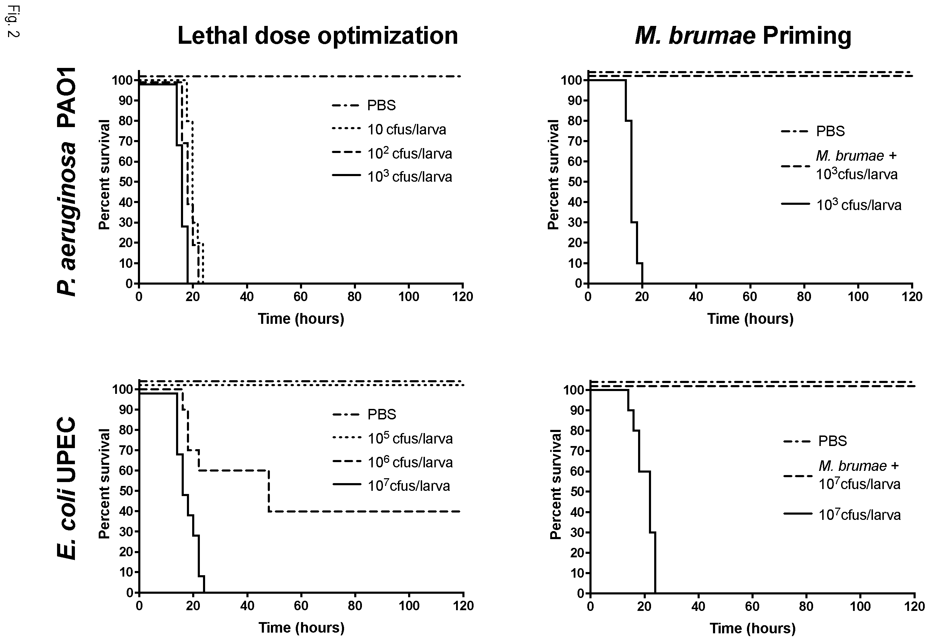

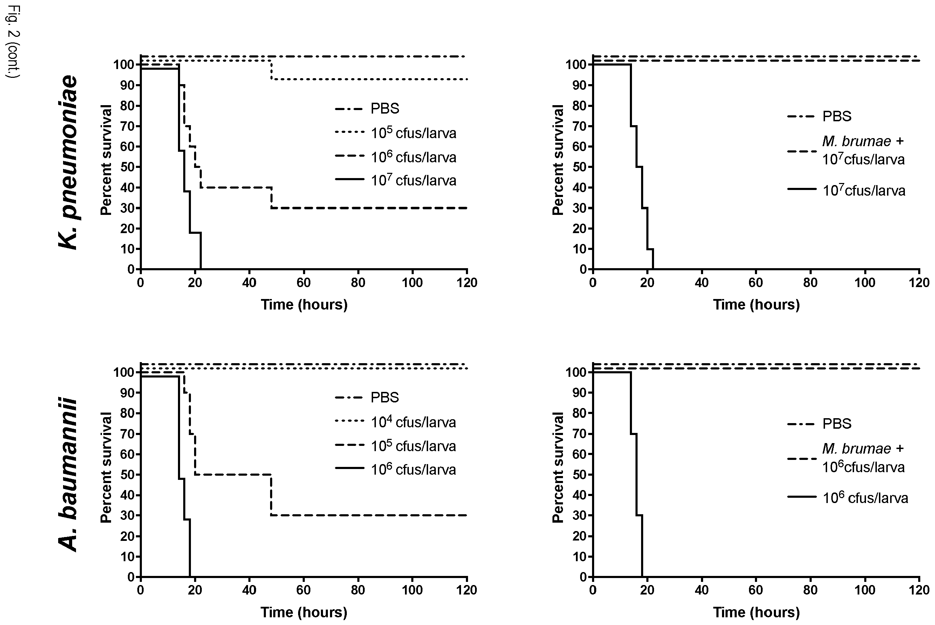

**Figure 2****: Kaplan Meier survival curves of *G. mellonella* larvae infected with highly virulent and antibiotic-resistant bacterial pathogens infections.** Bacterial lethal dose quantification for pathogens: *P. aeruginosa* PAO1, *E. coli* UPEC, *K. pneumoniae,* and *A. baumannii.* Effect of 24 h *M. brumae* priming treatment in larvae infected later (n=30) with lethal doses of bacterial pathogens.

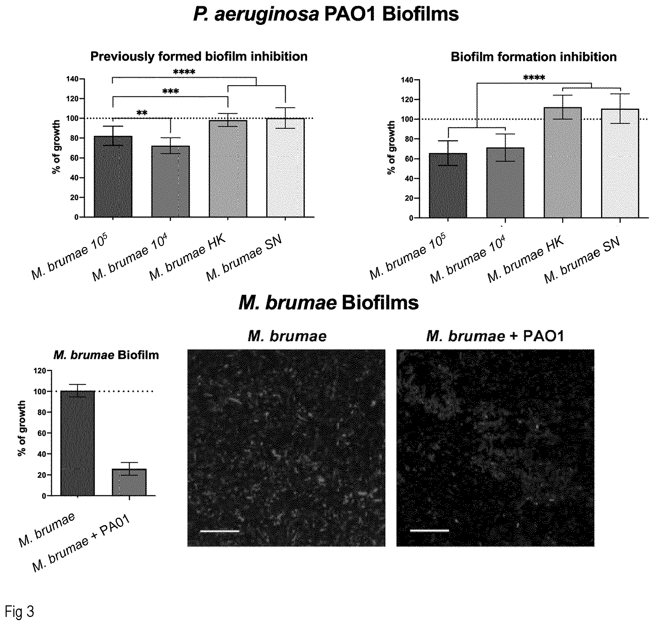

**Figure 3****:** Effect of different concentrations of *M. brumae* (10⁴, 10⁵ CFUs), heat-killed *M. brumae* (HK), and supernatant (SN) on *P. aeruginosa* PAO1 biofilms; previously formed (72 h mature biofilms) and co-cultivated simultaneously with *M. brumae* from the initiation of biofilm formation. Optimum growth, 100%, is considered for *P. aeruginosa* PAO1 biofilms developed without any treatment (dotted line). Data are presented as the mean values ± SD of three independent experiment replicates, statistical differences were determined using One-Way ANOVA, Tukey's multiple comparisons test: *****p <* 0,0001, *** *p* < 0,001, ** *p* < 0,01.

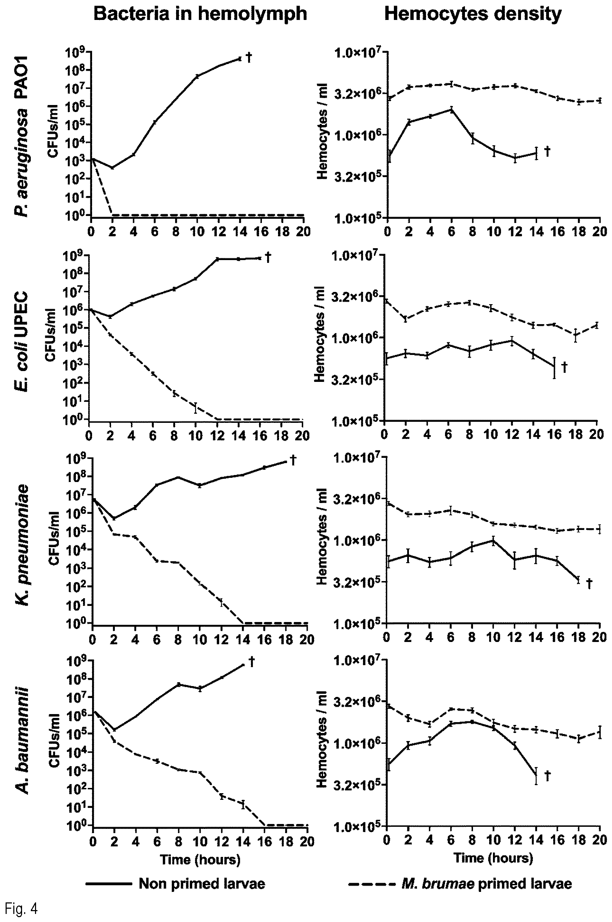

**Figure 4****: *M. brumae* primed or non-primed larvae regarding the progression of lethal bacterial pathogens infections.** The CFUs/ml and the concentration of hemocytes in hemolymph were quantified every 2 h in *G. mellonella* larvae; *M. brumae* primed 24 h prior to pathogens injection (solid lines) or non-primed (dashed line) after infection with lethal doses of pathogens: *P. aeruginosa* PAO1, *E. coli* UPEC, *K. pneumoniae* and *A. baumannii.* The dagger symbol (†) indicates the time point at which 100% of the larvae died.

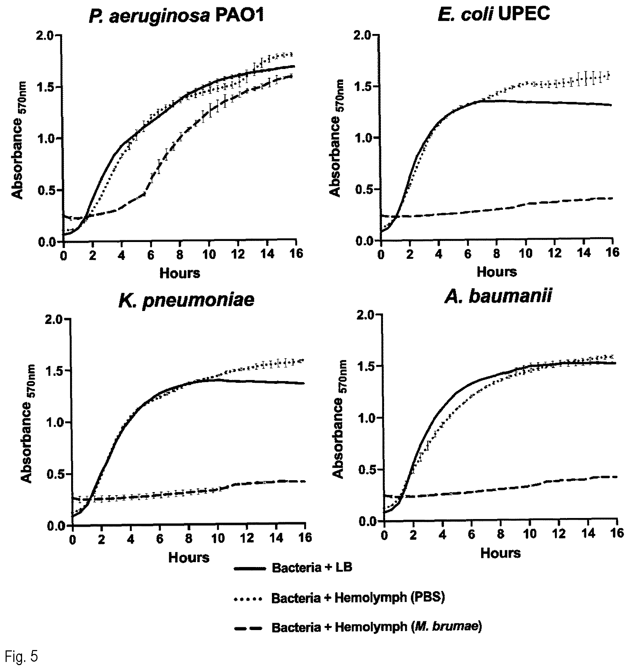

**Figure 5****: Characterization of isolated hemolymph without hemocytes from *G*. *mellonella* larvae, primed with** *M.* ***brumae* (24 h) or non-primed, against pathogenic bacteria.** Growth kinetics of pathogenic bacteria in LB broth mixed with hemocyte-free hemolymph (1:1 volume), absorbance at OD₅₇₀ₙₘ was measured every 15 min during 16 h. Hemocyte-free hemolymph contains immune-relevant molecules; antimicrobial peptides (AMP)s, enzymes, and proteins, among other compounds (lipids, sugars, water...)

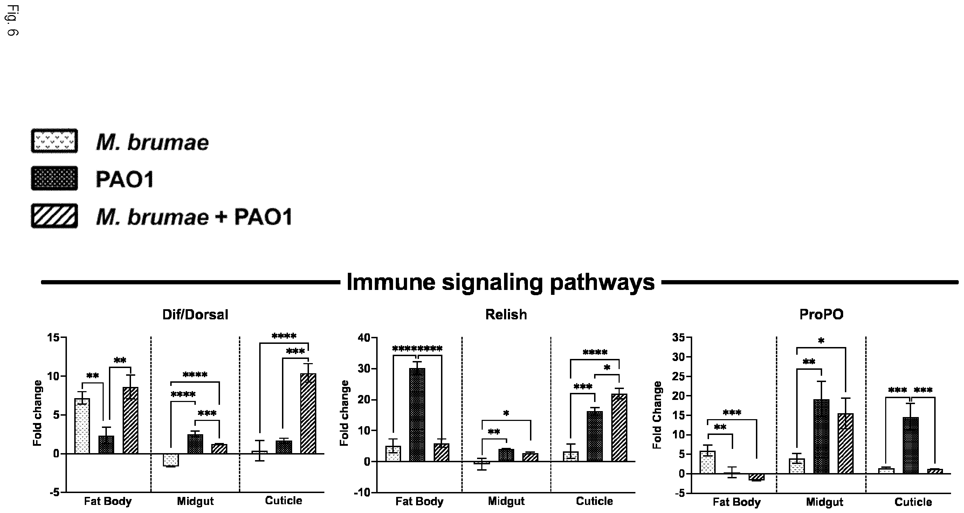

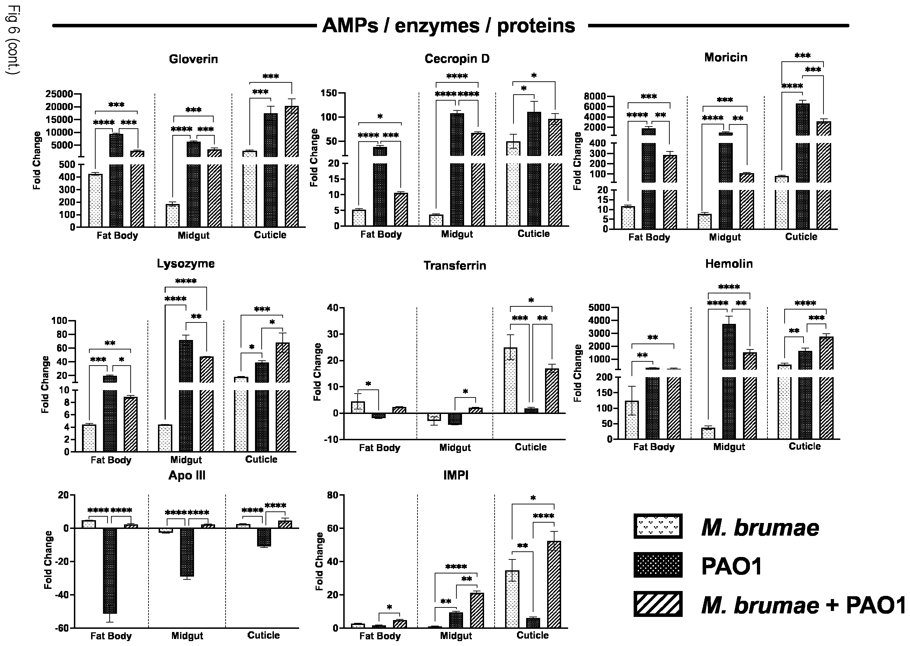

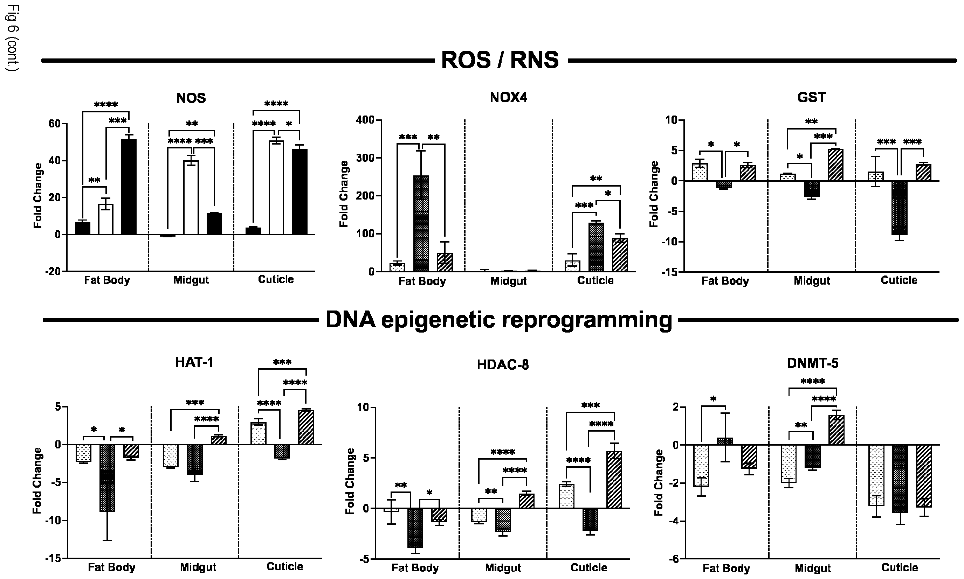

**Figure 6****: Genetic expression patterns of *G*. *mellonella* larvae in response to *M. brumae, P. aeruginosa* PA01 and priming (*M, brumae* and PAO1) injections focused on relevant organs and tissues.** RT-PCR analysis of different gene expression levels involved in relevant immune response activities in the fat body, midgut and cuticle of larvae. **Immune signaling pathways:** Dif/Dorsal (dorsal-related immunity factor, related with toll pathogen recognition pathway), Relish (related with Imd pathogen recognition pathway) and ProPO (Prophenoloxidase enzyme crucial in melanization process). **AMPs, enzymes, and proteins:** Gloverin, cecropin D and moricin are necessary AMPs against bacteria. Lysozyme (degrades bacterial cell wall), transferrin (sequesters iron, relevant to bacterial pathogenicity) and hemolin (associated with pathogen recognition and cellular immune response) are enzymes. Apo III (apolipophorin III, multi-functions, involved in phagocytosis and clotting) and IMPI (inducible metalloproteinase inhibitor) are relevant proteins. **ROS and RNS** (reactive oxygen and nitrogen species): NOS (Nitric oxide synthase) and NOX4 (NADPH oxidase) are pro-oxidative enzymes that generate ROS and RNS to kill pathogens; GST (Glutathione S-transferase) is a detoxification enzyme that protects cells from oxidative stress. **DNA epigenetic reprogramming:** HAT-1 (histone acetyltransferase 1) and HDAC-8 (histone deacetylase 8) regulate DNA acetylation/deacetylation, and DNMT-5 (DNA methyltransferase 5) regulates DNA methylation.

### Detailed description of the invention

All terms as used in this disclosure, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

*Mycobacterium brumae* (also known as *Mycolicibacterium brumae)* is a rapidly growing, non-pathogenic mycobacterial species originally isolated from environmental and human samples. In a particular embodiment of the present disclosure, the *M. brumae* is *Mycobacterium brumae* (ATCC 51384^{T}). However, the *M. brumae* strain for use as described in the present disclosure is not limited to any specific *M. brumae* strain.

Mycobacteria and, in particular, *M. bovis* BCG, have been previously disclosed as adjuvants to boost the effect of the active agents contained in vaccines. However, it has now been surprisingly found that this particular *Mycobacterium, M. brumae,* may completely prevent infection of ESKAPEE pathogens on its own, i.e., as sole active agent. As shown in the examples below, while *M. bovis* BCG may have some preventive effect when low doses of pathogens are inoculated in the animal model, *M. brumae* completely prevents infection even at lethal doses of the pathogens. The superior protective effect of *M. brumae* is particularly apparent when compared with other non-pathogenic mycobacteria, such as *M. smegmatis.*

The present disclosure is thus related to *M. brumae* or a composition thereof, for preventing or treating infections, as well as for preventing or treating the diseases caused by such infections.

It is envisaged that the *M. brumae* of the first to third aspects may be used in the form of various fractions common in the art, including but not limited to viable cells, non-viable cells, attenuated cells (i.e. viable but non-metabolically active cells), cell lysates, cell homogenates, cell-free extracts, and even active supernantants. It is also envisaged to use specific components of *M. brumae.* Therefore, the first to third aspects of the present invention not only contemplate *M. brumae* as whole cells, but also as a cell lysate, a cell-free extract, a component, or a culture supernantant of *M. brumae,* for use in the prevention and/or treatment of an infection. When contemplating *M. brumae* whole cells, said cells can be viable cells, non-viable cells, or attenuated cells (i.e. viable but non-metabolically active cells). However, it may be advantageous to use viable cells of *M. brumae,* since they provide a higher protective effect against the pathogens. For example, as shown in the examples below, viable *M. brumae* may slow down pathogen biofilm formation. Therefore, in one particular embodiment, the *M. brumae* of the first to third aspects is viable.

In one embodiment, the use is for preventing or treating infections, or the diseases caused by the infections, in an animal, in particular in higher animals. In particular embodiments, the use is for prevention or treatment in fish. In another embodiment, the use is for prevention or treatment in mammals, including humans.

In one embodiment of the first to third aspects of the invention, *Mycobacterium brumae* is the sole active ingredient. This means that *M. brumae* is by itself responsible for preventing the infection. In other words, in one embodiment, *M. brumae* is for use in the prevention and/or treatment of the infection in the absence of further active ingredients. Another embodiment is directed to *M. brumae* for use in the prevention and/or treatment of an infection, wherein the *M. brumae* is not used as an adjuvant. Another embodiment of the first to third aspects refers to a composition consisting essentially of *M. brumae* together with acceptable excipients and/or carriers. In another embodiment of the first to third aspects, the composition consists of *M. brumae* together with acceptable excipients and/or carriers.

In another embodiment of the first to third aspects, *M. brumae* is administered in a preventively or therapeutically effective amount. The expression "preventively or therapeutically effective amount" as used herein, refers to the amount of *M. brumae* that, when administered, is sufficient to prevent the infection partially or totally, or to treat or alleviate the disease caused by the infection partially or totally. The particular dose of *M. brumae* administered according to this invention will of course be determined by the particular circumstances surrounding the case, including the route of administration, the particular infection addressed, the subject, and similar considerations. In one embodiment, the preventively or therapeutically effective amount of *Mycobacterium brumae* is from 10¹ to 10¹² cells. In another embodiment, the preventively or therapeutically effective amount of *Mycobacterium brumae* is from 10⁴ to 10¹² cells. In another embodiment, the preventively or therapeutically effective amount of *Mycobacterium brumae* is from 10⁶ to 10¹² cells. In a particular embodiment, the preventively or therapeutically effective amount is from 10⁷ to 10¹¹ cells, for example 10⁹.

In one embodiment of the third aspect, the composition of the present disclosure is a pharmaceutical composition which comprises *M. brumae* together with pharmaceutically acceptable carriers or excipients. The expression "pharmaceutical composition" encompasses both compositions intended for human as well as for non-human animals (i.e. veterinarian compositions). The expression "pharmaceutically acceptable carriers or excipients" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, suspending agents, stabilizing agents, and/or oils. Excipients such as coloring agents, coating agents, sweetening, and flavouring agents can be present in the composition, according to the judgment of the formulator.

The pharmaceutical compositions of the invention can be presented in any dosage form, for example, solid or liquid, and can be administered by any suitable route, for example, oral, parenteral, rectal, intravaginal, intravesical, topical, intranasal or sublingual route, for which they will include the pharmaceutically acceptable excipients necessary for the formulation of the desired dosage form, for example, topical formulations (ointment, creams, lipogel, hydrogel, etc.), eye drops, aerosol sprays, injectable solutions, osmotic pumps, etc. *Mycobacterium brumae* can be formulated in liquid suspensions or solid forms. Liquid preparations can be solutions for suspension or emulsion in aqueous solutions (water, saline or phosphate-buffered saline (PBS), non-aqueous solutions or both (aqueous suspensions, oil emulsions, water-in-oil emulsions or oil-in-water emulsions, microemulsions, nanoemulsions or liposomes).

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, water, saline, sucrose, mannose, trehalose, sodium glutamate, glycerol, olive, sesame and soybean oils, and non-ionic and ionic polymers such as polyoxyethylenesorbitan monooleate (Tween), hyaluronic acid and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding excipients include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

*M. brumae* may be administered to the patient via the parenteral, oral, sublingual, nasal, pulmonary, rectal, intravaginal, intravesical, topical, optical, or otic route. In a preferred embodiment, *M. brumae* is administered via a parenteral route selected from subcutaneous, intradermal, subdermal, intraperitoneal, or intravenous injection.

In one embodiment the pharmaceutical composition of the third aspect is a parenteral composition, an intradermal composition, a transdermal composition, an intravenous composition, a subcutaneous composition, or a composition for application to the mucosa, (sublingual, oral, intestinal, nasopharyngeal, pulmonar, vaginal, vesical, or rectal).

The present disclosure also contemplates the use of compositions comprising *M. brumae* which are not pharmaceutical compositions. For example, food or feed additives, food or feeds, and nutraceutical compositions comprising *M. brumae* are also contemplated for use in preventing or treating infections in the sense of the present disclosure. These compositions may further comprise additional acceptable ingredients, such as excipients and/or carriers which are commonly known in food or feed compositions. The disclosure also contemplates *M. brumae* for use as a probiotic (for preventing or treating infections in the sense of the present disclosure).

In particular embodiments, *M. brumae* or compositions thereof as described above are for use in preventing infections, or for use in preventing a disease caused by an infection, or for use in preventing a disease caused by a pathogen. As used herein, the term "preventing" includes any of following: the prevention of the infection, disease, disorder, or of one or more symptoms associated with the infection, disease, or disorder; a reduction or prevention of the development or progression of the infection, disease, disorder, or symptoms. The term "prevention" can also be worded as "prophylactic treatment" or "preventive treatment". In other words, the disclosure refers to *M. brumae* for protecting a subject against infections and diseases caused by said infections. The pharmaceutical compositions of the invention may also be considered as vaccines. Thus, the disclosure contemplates a vaccine comprising a therapeutically effective amount of an *M. brumae* or pharmaceutical composition thereof as defined above. The disclosure also contemplates use of *M. brumae* or pharmaceutical composition thereof as defined above for the preparation of a vaccine.

In other embodiments, *M. brumae* or compositions thereof as described above is for use in treating infections, or for use in treating a disease caused by an infection, or for use in treating a disease caused by a pathogen.

As used herein, the term "treatment" contemplates the reduction or elimination of an existing infection, disease, disorder, or symptoms.

In the sense of the present disclosure, the terms "preventing" and "treating" are not understood as absolute. This means that prevention or treatment of the infection may be partial but, in any case, enough to prevent the subject from suffering from the associated disease or to suffer a milder form of the disease.

In one embodiment of the first to third aspects, the infection is caused by a bacterium. In one embodiment of the first to third aspects, the infection is caused by an antibiotic-resistant bacterium. In particular embodiments, the infection is caused by a nosocomial pathogen. In other particular embodiments the infection is caused by a pathogen selected from the group consisting of *Pseudomonas aeruginosa, Klebsiella pneumoniae, Salmonella, Vibrio, Mycobacterium, Escherichia coli, Streptococcus, Staphylococcus, Haemophilus, Enterococcus, Clostridium, Neisseria, Helicobacter, Listeria, Chlamydia* and *Acinetobacter baumannii,* In more particular embodiments the infection is caused by a ESKAPEE pathogen, i.e., the infection is caused by a pathogen selected from the group consisting of *Enterococcus faecium, Staphylococcus aureus, Klebsiella pneumoniae, Acinetobacter baumannii, Pseudomonas aeruginosa, Enterobacter spp, Escherichia coli,* or combinations thereof. More in particular, the infection is one caused by a bacterium selected from the group consisting of *Pseudomonas aeruginosa, Klebsiella pneumoniae, Escherichia coli, Acinetobacter baumannii, and combinations thereof.*

The diseases caused by the infections (hence addressed by the present disclosure) are typically skin infections; eye, ear and sinus infections; respiratory tract infections; oral and gastrointestinal infections; genital and urinary tract infections; blood (septicemia) and central nervous infections (encephalitis and meningitis). The previous list is non-exhaustive and does not exclude other diseases caused by pathogenic infections.

In some embodiments, the pharmaceutical composition as defined above may optionally further comprise an adjuvant. An "adjuvant" is an agent that acts in a non-specific manner to increase the therapeutic response. In another more particular embodiment, the adjuvant is selected the group consisting of alum, aluminium hydroxide, aluminium phosphate, Freund's complete adjuvant, squalene, monophosphoryl lipid A, the two-component liposomal adjuvant system CAF01, CpG ODN 1018, QS-21, lipid nanoparticles, and combinations thereof.

In some embodiments, *M. brumae* or the pharmaceutical composition comprising the same may be used in combination with an adjuvant in any of the uses as defined above. Although *M. brumae* may be used on its own, as sole active ingredient, in other embodiments, *M. brumae* or the composition comprising the same may be used in combination with a further active agent in any of the uses as defined above. In particular embodiments, the combined use comprises simultaneous, sequential or separate administration within a therapeutic interval of the *M. brumae* or the composition comprising *M. brumae* and the adjuvant or further active agent. The further active agent may be selected from the group consisting of antibiotics (antibacterials, antifungals, antiparasitics), antivirals, antiseptics, antimicrobial proteins or peptides, bacteriophages, probiotics, natural compounds such as plant-extracts or essential oils, nanomaterials, anti-toxins, immune modulators (anti-microbial antibodies, immune activator agents, immune-checkpoint inhibitors, biologics, corticosteroids), and combinations thereof. Combination with any of the further active agents may potentiate the activity of *M. brumae.* All embodiments defined above for the use of *M. brumae* or for the compositions also apply to the combined therapy.

As mentioned above, *M. brumae* may be used in any suitable form, such as viable cells, non-viable cells, attenuated cells (i.e. viable but non-metabolically active cells), cell lysates, cell homogenates, cell-free extracts, active supernantants, or specific components of *M. brumae* which are capable of eliciting an antipathogenic response in the subject. All of these are obtainable by methods well known in the art.

*M. brumae* may be obtained by a process of preparing a viable bacterial cell suspension that comprises: (i) inoculating *M. brumae* in a culture medium, (ii) subjecting the inoculated culture medium of the step (i) to conditions suitable for growth of *M. brumae,* and (iii) optionally subjecting the medium resulting from step (ii) to a concentration step.

Non-limiting conditions suitable for the growth of *M. brumae* may be as follows. *M. brumae* can be cultured on a substrate that may be a liquid broth or solid culture medium using standard culture medium such as Sauton or Middlebrook media. In particular embodiments *M. brumae* is grown in Middlebrook medium, for example Middlebrook 7H10 or 7H11 agar, or Middlebrook 7H9 broth. The medium may be supplemented with oleic acid-albumin-dextrose-catalase. Suitable conditions for the growth of *M. brumae* are temperatures of around 37°C, time of around 1 week, and aerobic conditions. Bacterial grown on the surface of solid culture media may be scraped and resuspended, for example, in phosphate-buffered saline to obtain a cell suspension of desired cell density. Bacteria grown in liquid broth may be collected by centrifugation and also resuspended in an appropriate buffer, such as phosphate-buffered saline. An example of the detailed procedure for obtaining cells of the bacteria of the invention in liquid medium is set forth in Example 1.

The cell suspension may then be used directly, resuspended to a desired density, subjected to dehydration or lyophilization. Cryoprotectants and/or lyophilization additives are often added. Washing steps may be introduced when required.

The cells may also be frozen. Preservatives such as glycerol are often used when freezing in order to avoid damage to the bacterial cells. The cells may also be frozen or stored in porous beads for microbiological culture preservation

The culture's supernatant contains metabolites secreted by the bacterial cells during their growth which may have antipathogenic effects. Said metabolites may be identified, for example, by HPLC. Said active supernatant may be obtained when removing the bacterial cells from the culture medium after growing *M. brumae.* Moreover, the present disclosure also contemplates using the *M. brumae* cells in a disrupted form, for example, lysed bacterial cells, or even a cell free extract resulting from disrupting the cells and removing cell wall and cell membrane components. Cells may be disrupted by any suitable means, such as by using a French press, sonication or incubation with lysozyme. Particular fractions of components of the cells that show good activity may be isolated by protein, lipid and polysaccharides purifications, liquid and thin layer chromatographic techniques, differential centrifugation, etc. The culture's supernatant, cell free extract, fraction or component may be further concentrated, for example by ultrafiltration or centrifugation, to enrich said medium or extract in desired metabolites.

Non-viable cells of *M. brumae* may be obtained by phenolization, heat treatment, irradiation and formalin or formaldehyde treatment.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

The larvae from the greater wax moth, *Galleria mellonella,* stands out as an ideal model for studying responses against pathogens, reporting results comparatively to vertebrates (Ménard 2021).

The following examples show the priming capacity of *M. brumae,* whose pre-injection demonstrates a 100% protective effect against lethal doses of highly virulent and pathogenic bacterial species in *G. mellonella..* Importantly, the inventors have found that the response elicited by *M. brumae* in the treated animals is related to increased levels of AMPs, enzymes, and antibacterial compounds, as well as changes in the phagocytic capacity in primed and non-primed larvae. Without wanting to be bound by theory, this suggests that *M. brumae* elicits multiple response mechanisms in the treated animals that work synergically to effectively protect said animal against the pathogenic infection.

### 1. Material and Methods

### 1.1. Bacterial strains, plasmids, and maintenance

*M. brumae* (ATCC 51384^{T}) was transformed with the plasmid pETS218 (constitutive expression of the green fluorescence protein-GFP) as previously reported (Campo-Pérez 2021) and used in all the G. *mellonella* infections and confocal microscopy cellular response experiments. *M. brumae* was also transformed with the plasmid pCherry10 (Carroll 2010) (constitutive expression of the red fluorescent protein-mCherry) used in anti-biofilm assay images and fluorescence quantifications. Both *M. brumae* transformed strains were grown in Middlebrook 7H10 agar medium (Becton Dickinson, New Jersey, USA) supplemented with 10% Oleic-Acid-Dextrose-Catalase (OADC) enrichment and specifically supplemented with 50 µg/mL kanamycin for *M. brumae* pETS218 or 100 µg/mLhygromycin for *M. brumae* pCherry10 as selection markers. *M. smegmatis* (ATCC 700084) and *M. bovis* BCG were cultured in Middlebrook 7H10 agar plates without antibiotics.

Pathogenic *M. abscessus* DSMZ 44196 (ATCC 19977) original smooth (S) morphotype and a rough (R) variant previously obtained as a natural mutant of the wild-type strain (Brambilla 2016) were grown on Tryptic Soy Agar (TSA, Sharlau, Barcelona, Spain). *B. thuringiensis* (ATCC 10792) was cultured also in TSA.

*P. aeruginosa* PAO1 (ATCC 15692) reference laboratory strain, previously transformed with a plasmid (pETS130lux) expressing a constitutive luciferase gene cassette (*lux*) as a bioluminescent bioreporter (Moya-Andérico 2020), was used in all *G. mellonella* larvae infections, grow kinetics and biofilm assays quantifications. Also, *P. aeruginosa* PAO1 with an eGFP (miniTn7) vector inserted in its chromosome (Klausen 2003) was used for phagocytosis quantification studies. Both strains were cultured in Luria-Bertani broth (LB, Sharlau, Barcelona, Spain) supplemented with gentamycin 50 µg/mL in overnight (ON) cultures.

The non-pathogenic *E. coli* K12 substrain MG1655 (ATCC 700926), the uropathogenic (UPEC) *E*. *coli* CFT073 (ATCC 700928), *K. pneumoniae* (ATCC 13883) and *A*. *baumannii* (ATCC 19606) were cultured in LB broth.

### 1.2 Bacterial injection inoculum preparation

Mycobacteria strains were grown on 7H10 plates for 7-11 days and used to prepare the injection suspensions. A loopful of isolated colonies was scraped from the plates and placed in a sterile 10 mL glass test tube (SciLabware, Stoke-on-Trent, UK) with 5 mm diameter glass beads (dDBiolab, Barcelona, Spain) for disaggregation by vortexing (around 1 min). The mycobacteria were then resuspended in phosphate-buffered saline (1x PBS 7.4 pH) (Fisher Scientific, Madrid, Spain) by vortexing and adjusted to a 1.0 McFarland turbidity standard to obtain the different concentrations required in each experiment.

For all other bacterial strains, inoculum preparation was performed from ON cultures. Bacterial broth cultures were centrifuged at 4000 *g* for 10 min, the supernatants were discarded, and the pellets were resuspended in 1x PBS. This washing was performed three consecutive times to remove the original culture medium completely. Then, the optical density of bacterial suspensions (OD_{590 nm}) was measured using a 1/10 dilution and adjusted to a final OD_{590 nm} of 1. From these suspensions, 1/10 serial dilutions in PBS were made to obtain the desired concentration for each *G. mellonella* infection assay. All the bacterial inoculum suspensions were properly diluted and seeded on agar plates to confirm bacterial quantification by colony forming units (CFUs) recounts.

### 1.3. Galleria mellonella maintenance, infection, and survival curves

*G. mellonella* larvae were fed *at libitum* with an artificial diet based on 15 % corn flour, 15 % wheat flour, 15 % infant cereal powder, 11 % powdered milk, 6 % brewer's yeast, 25 % honey and 13 % glycerol. The larvae were kept at 34°C in a dark environment until they reached an optimum size (around 200 mg). *G. mellonella* larvae were injected in all the experiments with 10 µl of bacterial inoculum, containing the desired bacterial counts for each experiment, through the top right proleg using a 25 µl Hamilton microsyringe (Hamilton, Reno, USA).

For all the priming experiments, the larvae were pre-injected with the different bacterial suspensions (primed) or 1x PBS (non-primed) 24 h before bacterial infection with the lethal concentrations of the different pathogens, using the second top right proleg. Priming doses for each bacterium used in the screening experiments were previously optimized (data not shown), ensuring the safety and survival of all injected larvae. On this premise, priming doses were: 10⁴ CFUs/larvae for *M. brumae* (live and heat-killed), *M. abscessus* R / S, *M. bovis* BCG, and 10⁵ CFUs/larvae for *M. smegmatis, E. coli* MG1655 and *B. thuringiensis.* The pathogenic *P. aeruginosa* PAO1 was lethal in less than 24 h in all concentrations tested, but doses 10, 10² and 10³ CFUs/larvae were selected as low, medium, and high lethal doses, to determine the priming effect variations facing different pathogen concentrations.

Lethal doses for *G. mellonella* larvae of the pathogens: *E. coli* UPEC, *K. pneumoniae* (both 10⁷ CFUs/larvae) and *A. baumannii* (10⁶ CFUs/larvae) were determined by injecting different doses until achieving 100% lethality. In all the experiments, larvae were kept at 37_{°}C during the infection course and survival was monitored each 2 h between 12 h and 24 h post-infection period (the highest mortality is recorded in this period) and subsequently only once a day. Dead larvae were considered those that remain immobile under physical stimuli, usually accompanied by a whole larvae's dark color caused by melanization.

### 1.4. M. brumae anti-P. aeruginosa biofilm assays

For evaluating the anti-biofilm activity assays, ON cultures of *P. aeruginosa* PAO1 carrying the pETS130lux were adjusted to an OD_{550 nm} of 0.1 in TSB + 0.2% glucose (Panreac Quimica, Barcelona, Spain). Then, 200 µL/well of bacterial suspension was added to Costar^{®} 96-well black polystyrene plates. Plates were cultured at 37°C with humidity saturation conditions to allow biofilm formation on the walls of the wells. After 72 h, planktonic bacteria were removed by washing three times (3x) with 300 µL/well of 1x PBS to keep exclusively biofilm-forming bacteria. Once mature *P. aeruginosa* PAO1 biofilms were established, they were treated with 200 µL suspensions of live *M. brumae* (10⁴ CFUs/well and 10⁵ CFUs/well determined using a 1 McF turbidity standard), heat-killed (121°C, 21 min) and the supernatant was included. After 24 h treatment, the wells content was removed and washed 3x with PBS as previously described to remove planktonic cells. After the last wash, the remaining biofilm formed in each well was homogenized entirely in PBS, applying strong pipetting. Finally, each well level of *lux* expression (*P. aeruginosa* cells) was quantified using a Spark^{®} multimode microplate reader (TECAN, Männedorf, Switzerland).

In the simultaneous co-cultivation experiments (biofilm formation inhibition), *P. aeruginosa* PAO1 suspensions were grown simultaneously with *M. brumae* live bacteria / heat-killed / supernatants for 72 h, and *lux* expression was measured as previously described.

### 1.5. P. aeruginosa PAO1 anti-M. brumae biofilm assays

Transformed *M. brumae* with pCherry10 plasmid strain was cultured for 3 days at 37°C, 200 rpm in 7H9 broth medium supplemented with 10% ADC, 0.05% Tween 80 and 100 µg/mL hygromycin. To minimize the clumping effect of the mycobacteria, Erlenmeyer flasks containing 5 mm glass beads were used. This broth culture was adjusted to an McF 1 suspension (5×10⁶ CFU/mL) and grown in 7H9 medium + 0.2% glucose in Costar^{®} 96-well black polystyrene plates (200 mL/well) at 37°C and saturation humidity conditions for 120 h to allow mycobacterial biofilm formation. Then, 3x washes with PBS were carried out to remove both *M. brumae* bacterial cells in suspension and non-biofilm-forming precipitates. Subsequently, the suspension of *P. aeruginosa* PAO1 was added into the *M. brumae* biofilm-developed wells at an OD_{550 nm}= 0.1 for 72h. Finally, the biofilms were homogenized in PBS and quantified by measuring the mCherry expression associated with *M. brumae* using a Spark^{®} multimode microplate reader.

Confocal microscope images were performed from the *P. aeruginosa* PAO1 and *M. brumae* mCherry biofilms developed in the 96-wells plates. Biofilms were collected and stained with 4', 6-Diamidino-2-Phenylindole (DAPI) to stain all bacteria in the biofilm. *M. brumae* bacilli were detected distinctly by mCherry expression in red. Representative images were obtained in a Zeiss LSM 800 confocal laser scanning microscope (CSLM) using the 63× / 1.4 oil objective.

### 1.6. Isolation of hemolymph, seeding of bacterial CFUs and hemocytes recounts

To characterize the infection progression and *M. brumae* priming protective effect against highly virulent pathogens: *P. aeruginosa* PAO1, *E. coli* UPEC, *K. pneumoniae* and *A. baumannii,* primed and non-primed larvae were infected with lethal doses of each bacterial species tested. Every 2 h after infection, larvae hemolymph was isolated to quantify the bacterial load and the hemocyte density. For this, ten larvae of each infection condition were individually transferred into 1.5 mL Eppendorf tubes and anesthetized on ice for 10 min. Subsequently, the anal proleg of the anesthetized larvae was cut with a surgical blade to collect the hemolymph in the Eppendorf tube. The hemolymph of the ten larvae from the same infection condition was pooled. 100 µL were appropriately diluted (if necessary) and plated on LB agar plates for CFUs counting and bacterial load quantifications. Another 20 µL were diluted 1/2 in trypan blue (Sigma-Aldrich, St. Louis, USA) and loaded in a Neubauer chamber where hemocytes were counted, and its concentration was calculated using an inverted fluorescence microscope ECLIPSE Ti-S/L100 (Nikon, Tokyo, Japan). These processes were conducted every 2 h for 20 h or until 100% of the larvae died because of infection.

### 1.7. Co-culture of bacteria and hemocytes free hemolymph: agar plates and growth kinetics

The direct impact of hemolymph from larvae pre-exposed to *M. brumae* (primed) or PBS (non-primed) on highly virulent bacteria was evaluated. For this, one hundred larvae were infected with 10⁴ CFUs/larvae of *M. brumae* (priming dose) and another one hundred larvae with PBS (non-priming condition). After 24 h, the larvae hemolymph of the two conditions was isolated and pooled as described in the previous section, at this point, 0.6 mg/mL of L-cysteine (Sigma-Aldrich, St. Louis, USA) was added to the hemolymph to prevent melanization as previously reported (Admella 2022). To remove the hemocytes from the hemolymph and leave only circulating elements such as: AMPs, enzymes, proteins and different compounds, the hemolymph was centrifuged at 500 g, 10 min, 4°C and the supernatant was transferred to a new 1.5 ml Eppendorf tube, this process was repeated three times to ensure complete hemocyte removal.
For the hemolymph tests in solid agar cultures, the pathogenic bacteria: *P. aeruginosa, E. coli* UPEC, K. *pneumoniae,* and *A. baumannii* were grown in a bacterial lawn in LB agar plates using sterile microbiology swabs (dDBiolab, Barcelona, Spain) moistened in bacteria ON cultures adjusted to an OD_{550 nm} of 1.0. To evaluate possible growth inhibitory effects, three volumes drop of hemolymph free of hemocytes: 5, 10 and 20 µL, recently sown from both *M. brumae* primed and non-primed (PBS), were added on the plates. The plates were incubated for 24 h at 37°C, and the pathogen's growth in the areas occupied by hemolymph drops was evaluated.

Growth kinetics curves were performed to evaluate the effect of hemolymph on the growth of virulent bacteria. ON cultures in LB broth of the pathogenic bacteria were prepared and adjusted to an OD_{550 nm} of 0.1. Costar^{®} 96-well clear polystyrene plates (Corning, New York, USA) were filled with 100 µl/well of bacterial suspension for control without hemolymph treatment. The test wells were filled with bacterial 0.1 OD_{550 nm} suspension and hemolymph free of hemocytes in a 1:1 ratio (50 µl of bacteria and 50 µl of hemolymph). Control wells with 100 µl of primed (*M*. brumae-injected) and non-primed (PBS-injected) hemolymph were also used to subtract absorbance variations related to hemolymph melanization processes. Bacterial growth kinetics were performed using a Spark^{®} multimode microplate reader (Tecan, Männedorf, Switzerland) programmed to maintain a temperature of 37°C, 150 rpm agitation and to quantify absorbance (OD_{550 nm}) every 15 min during 16 h. After kinetics, the content of all wells was cultured on LB agar plates and grown at 37°C for 24 h to confirm the presence/absence of the tested pathogenic bacteria inoculated with primed / non-primed hemolymph.

### 1.8. Larvae dissection, RNA isolation and Real-time PCRs of relevant G. mellonella genes

Twenty *G. mellonella* larvae were injected with *M. brumae* 10⁴ CFUs/ml (priming dose), and another twenty with 1x PBS (non-primed). After 24 h, ten larvae pre-exposed to M. *brumae* and another ten injected with 1x PBS were infected with 10³CFUs/larva of *P. aeruginosa* PAO1. At this time, 4 groups of 10 larvae/each were distinguished: *M. brumae, M. brumae* + *P. aeruginosa* PAO1, PBS, and PBS + *P. aeruginosa* PAO1. After 14 h post-*P. aeruginosa* PAO1 infection, the larvae were snap frozen to facilitate the dissection process. Using a stereoscopic microscope, the larvae were dissected by making an anterior-posterior cut in the ventral part using a surgical blade. The adipose tissue, midgut, and cuticle of all the larvae were obtained and placed in 1.5 ml Eppendorf tubes on ice.

The tissues were immediately homogenized using RNase-free disposable pestles (Fisher Scientific, Waltham, USA), weighed, and 30 mg of each tissue taken from each of the four groups were used for RNA isolation. RNA extraction was performed following GeneJET RNA Purification Kit instructions (Thermo Fisher Scientific, Madrid, Spain). The RNA obtained was subjected to a treatment with 10x TURBO DNase (Life Technologies, Carlsbad, USA) for 1h to eliminate possible DNA remains. DNA absence test was performed through PCR amplification of 18S rRNA housekeeping gene, using genomic DNA of *G. mellonella* as a positive control. RNA obtained was quantified in an M200 PRO multimode microplate reader (TECAN, Männedorf, Switzerland). RNA to cDNA reverse-transcription was performed using Maxima Reverse Transcriptase (Thermo Fisher Scientific, Madrid, Spain) along with Oligo (dT)18 primers (Thermo Fisher Scientific, Madrid, Spain) by following the manufacturer's instructions. The cDNA obtained was stored at - 20°C until use. Quantitative real-time PCR (qRT-PCR) was performed using PowerUp^{™} SYBR^{™} Green Master Mix (Applied Biosystems, Foster City, CA, USA) in a StepOnePlus^{™} Real-Time PCR System (Applied Biosystems, Foster City, CA, USA) according to the manufacturer's protocol. All qRT-PCR reactions used specific primers of *G. mellonella* relevant genes listed in **Table 1.**

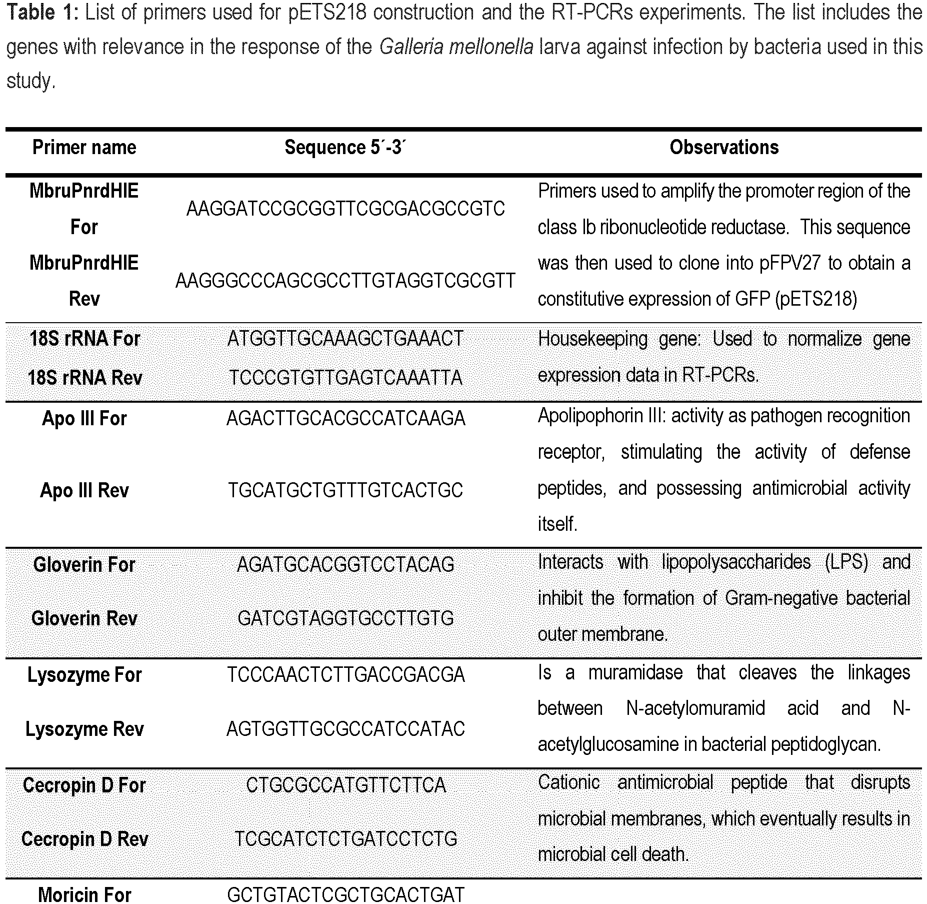

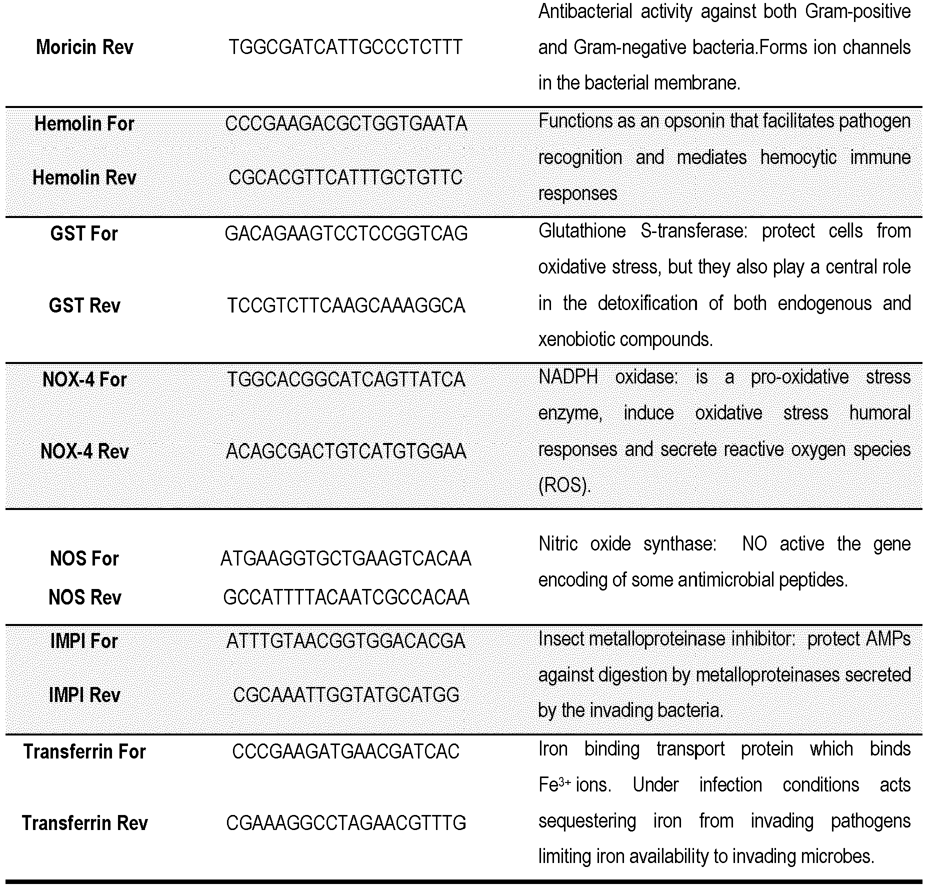

### 2. Results

### 2.1. Priming with mycobacteria trigger a protective effect against bacterial infections in G. mellonella larvae

Different mycobacteria, such as the non-pathogenic *M. brumae* and *M. smegmatis,* the attenuated *M. bovis* BCG, and the pathogenic *M. abscessus* R and S, were used to screen possible priming effects. As non-mycobacterial controls, the non-pathogenic *E. coli* MG1655 (Gram-negative bacterium) and *B. thuringiensis* (Gram-positive) were also selected. *P. aeruginosa* PAO1 was chosen as a pathogen due to its high pathogenicity at small doses and short times of *G. mellonella* larvae survival after infection.

As shown in **Figure 1A****,** survival curves show a clear protective effect in larvae pre-injected with mycobacteria. Specifically, survival is maintained at 100% when pre-exposing the larvae to *M. brumae* and both R and S morphotypes of *M. abscessus* regardless of the dose after *P. aeruginosa* was injected. In the larvae pre-injected with *M. smegmatis,* survival rates were close to 100% at low and medium infection concentrations of *P. aeruginosa* PAO1 (10 and 10² CFUs/larva), but survival rates dropped to 30% at high concentrations of *P. aeruginosa* PAO1 (10³ CFUs/larva). Survival rates dropped even further when priming with *M. bovis* BCG. The priming assays using *E. coli* MG1655 and *B. thuringiensis* do not show any improvement in larvae survival following similar curve patterns as non-primed larvae at the same *P. aeruginosa* PAO1 doses **(****Figure 1A****).**

The priming effect is time-dependent, and its effectiveness decreases over time. In this sense, when *P. aeruginosa* PAO1 lethal doses are injected 24, 48, or 72 h post-priming with *M. brumae,* the protection is total (100% survival). However, at 96 h post-priming, there is a 24 h delay in mortality, but all priming larvae die at 48 h **(****Figure 1B****).** It is also shown that using heat-killed *M. brumae* induces the protective priming effect, but with much less intensity than the live mycobacteria. Specifically, 80% of the larvae survive at the *P. aeruginosa* PAO1 low concentration (10 CFUs/larva), 40% at the medium (10² CFUs/larva), and only 5% at the high *P. aeruginosa* PAO1 concentration (10³ CFUs/larva).

Regarding homologous protection, using safe doses of *M. abscessus* R and S and subsequently lethal (10⁶ CFUs/larva) doses of the identical pathogenic mycobacteria, no protective effect was observed. Survival rates are lower in primed groups than in non-primed larvae at the same *M. abscessus* lethal doses **(****Figure 1B****).**

### 2.2. M. brumae priming protective effect is effective against different lethal virulent infections

The heterologous protective effect of *M*. *brumae* priming *G*. *mellonella* larvae was studied against other bacterial pathogens, such as *K. pneumoniae, E. coli* UPEC and *A*. *baumannii.* The lethal doses in all these bacteria were estimated by increasing the dose of injection until reaching 100% lethality. For *E. coli* UPEC and *K. pneumoniae,* lethal dose was 10⁷ CFUS/larva, while for *A*. *baumannii,* it was 10⁶ CFUs/larva **(****Figure 2****).** In all cases, the survival rates were dose-dependent and clearly higher for *P. aeruginosa* PAO1, which resulted in lethal conditions even injecting only 10 CFUs/larva.

Total protection was observed by priming the larvae with *M. brumae* (10⁴ CFUs/larva) and 24 h later with the previously estimated lethal doses of the pathogenic bacteria. 100% of the larvae primed with *M. brumae* survived the lethal dose (10 and 10² CFUs/larva) infections, while the control group (non-primed, PBS-injected) died in less than 24 h post-infection **(****Figure 2****).** This phenomenon was reported against all pathogens, regardless of their characteristics, indicating a generalized non-specific heterologous protection.

### 2.3. M. brumae slows down P. aeruginosa PAO1 biofilm development

Possible changes in the capacity to develop *P. aeruginosa* PAO1 biofilm were also characterized by co-cultivation with *M. brumae* cells. In this sense, co-cultivation with *M. brumae* caused a 20-30% decrease in the progression of mature *P. aeruginosa* PAO1 biofilms. This reduction was not reported using heat-killed M. *brumae* or *M. brumae* supernatant. When *M. brumae* and *P. aeruginosa* PAO1 were co-cultured simultaneously from the start of the biofilm formation, a 30-35% growth reduction of *P. aeruginosa* PAO1 was observed compared to *P. aeruginosa* PAO1 biofilms without treatment (Figure 3B). Again, the application of heat-killed *M. brumae* or supernatant did not affect the biofilm growth. Studying the involvement of *P. aeruginosa* PAO1 in an *M. brumae* mature biofilms, significant destruction of the biofilm and overrepresentation of *P. aeruginosa* PAO1 was observed, specifically, the presence of *M. brumae* cells in the biofilm was reduced by around an 80%. Confocal images clearly show a remodeling of the biofilm in which *P*. *aeruginosa* PAO1 prevails, replacing *M. brumae* (Figure 3B).

### 2.4. Priming larvae with M. brumae eliminate bacterial pathogens from hemolymph and induce overproduction of hemocytes

The progression of infection by *P. aeruginosa* PAO1, *E. coli* UPEC, *K. pneumoniae* and *A. baumannii* in *M. brumae*-priming and non-primed (PBS)-*G*. *mellonella* larvae were characterized by analyzing the concentration of bacterial pathogens and the density of hemocytes in hemolymph over time.

It was observed that priming larvae with *M. brumae* could eliminate pathogenic bacteria from their organism. Specifically, during the infection with *P. aeruginosa* PAO1 (10³ CFUs/larva), no bacteria were detected in the hemolymph of the *M. brumae*-primed larvae even at only 2 h post-infection, indicating an early elimination of the bacteria **(****Figure 4****).** In the case of *E. coli* UPEC, *K. pneumoniae* (10⁷ CFUs/larva) and *A. baumannii* (10⁶ CFUs/larva), a progressive reduction of the initial injected concentrations was observed in the *M. brumae-*primed larvae, until their complete elimination from the larvae hemolymph between 12 and 16 h post-infection **(****Figure 4****).** In infections in non-primed larvae, bacteria growth (*P*. *aeruginosa, E. coli, K. pneumoniae* and *A. baumannii*) in the hemolymph was observed until their death; specifically, 100% of the larvae dead between 14-18 h post-infection at concentrations above 10⁸ CFUs/ml in all cases. Regarding hemocyte concentration analysis, stable concentrations around 3×10⁶ hemocytes/mL were counted in all infected *M. brumae*-primed larvae throughout the 20 h study **(****Figure 4****).** However, in non-primed larvae, fluctuations in the number of hemocytes were observed throughout the progression of the different infections. In any case, concentrations remained in the order of 10⁵ hemocytes/mL, clearly lower than the reported in *M.* brumae-primed larvae. An induction in hemocytes production was reported in the first hours of infection with *P. aeruginosa* and A. *baumanni,* but over time the concentration decreased until the death of the larvae **(****Figure 4****).**

### 2.5. Hemocyte-free hemolymph isolated from M. brumaeprimed larvae prevents the growth of pathogenic bacteria

Once demonstrated *M.* brumae-primed larvae capacity to eliminate injected pathogenic bacteria from their hemolymph, the antibacterial activities of hemocyte-free hemolymph isolated outside the larval context were studied. The pathogenic bacteria such as *P. aeruginosa* PAO1, *E. coli* UPEC, *Kpneumoniae* and *A. baumannii* were grown on bacterial lawns in agar solid plates, and no restrictive effects on growth were observed in the area occupied by hemocyte-free hemolymph drops (results not shown), regardless of hemolymph was from *M. brumae*-primed or non-primed larvae. This demonstrates that hemocyte-free hemolymph has no inhibitory effect added in droplets on solid cultures against pathogen bacteria. However, when using hemocyte-free hemolymph in liquid culture kinetics, significant changes were observed in the growth dynamics of pathogenic bacteria. Hemocyte-free hemolymph from *M. brumae*-primed larvae completely inhibit the growth of *E. coli* UPEC, *K. pneumoniae,* and *A. baumannii* **(****Figure 5****),** the absence of growth was confirmed by plating the liquid cultures from the kinetics growth in LB agar plates, in which no CFU counts were reported. In the case of *P. aeruginosa* PAO1, the hemocyte-free hemolymph from *M.* brumae-primed larvae did not completely inhibit its growth, although its curve was modified concerning the control (LB) and the non-primed hemolymph (PBS), reducing its growth progression, especially during the first 6 hours of analysis **(****Figure 5****).** This result indicates that the AMPs, enzymes, proteins, and chemical compounds generated in the hemolymph of the larvae because of *M. brumae* priming could eliminate *E. coli* UPEC, *K. pneumoniae* and *A. baumannii* and slowing down but not completely inhibit the *P. aeruginosa* PAO1 progression.

### 2.6. Transcriptional expression patterns of relevant genes vary significantly between M. brumae-primed and non-primed infected G. mellonella in different larvae tissues.

The expression of different relevant genes was analyzed under four different conditions: *M. brumae* injection, *P. aeruginosa* PAO1 infection, primed-*M*. *brumae* larvae subsequently infected with *P. aeruginosa* PAO1, and PBS-injected larvae as a control on which the fold changes were established **(****Figure 6****).** Three relevant *G*. *mellonella* larvae tissues were selected: fat body, midgut, and cuticle, to distinguish more relevant gene patterns in each tissue.

A clear distinction was observed in genes related to induced signaling pathways: *M. brumae* and *M. brumae* + PAO1 induced a higher expression of the Dif/Dorsal gene (Toll signaling pathway), mainly in the fat body and cuticle, while *P. aeruginosa* PAO1 infection alone predominantly induced the Relish gene (Imd pathway). Regarding the prophenoloxidase (ProPO) gene involved in the melanization pathway, its expression was mainly induced in *P. aeruginosa* PAO1 infection alone in the larvae midgut and cuticle **(****Figure 6****).** The AMPs genes: gloverin, cecropin D and moricin were strongly induced in all tissues under all conditions tested, highlighting the condition in *P. aeruginosa* PAO1 alone in which the highest levels of AMPs overproduction were detected. Interestingly, it is also shown that the *M. brumae* priming induce the expression of AMPs, although to a lesser extent than those infected with PAO1 alone or those primed with *M. brumae* and subsequently infected with PAO1. The expression of the lysozyme enzyme gene (which degrades peptidoglycan of the bacterial cell wall) was especially transcriptionally induced in larvae infected by *P. aeruginosa* PAO1 and *M. brumae* + *P. aeruginosa* PAO1. However, clear gene expression inductions were also observed in *M. brumae*-primed larvae, especially in the cuticle of the larvae. Transferrin (iron ion sequestering enzyme) is specifically transcriptionally induced in the cuticle of *M. brumae*-primed larvae or *M.* brumae-primed and subsequently infected with *P. aeruginosa* PAO1. Hemolin (opsonin involved in phagocytosis) was strongly induced in all tissues, especially under conditions where PAO1 is infecting. The gene expression of the apolipophorin III protein (Apo III, multifunctional protein involved in pathogen recognition, phagocytosis, and AMP production) was very clearly transcriptionally repressed in all tissues, although in the fat body only when the larvae were infected with *P. aeruginosa* PAO1 alone, but interestingly not in the priming *M. brumae + P. aeruginosa* PAO1. The IMPI protein (inducible metalloproteinase inhibitor) gene was overexpressed mainly in the cuticle of larvae injected with *M. brumae* (both alone and with a subsequent PAO1 infection).

Enzymes related to producing reactive oxygen and nitrogen species (ROS / RNS), involved in eliminating pathogens, showed a pro-oxidative response in *P. aeruginosa* PAO1 infections. Increases of gene expression of NOS (Nitric oxide synthase) and NOX4 (NADPH oxidase) were clearly reported in fat body and cuticle accompanied by the repression of GST (Glutathione S-transferase) gene, an anti-oxidative enzyme slightly induced by *M. brumae* injection and repressed in PAO1 alone infections.

Finally, regarding the genes involved in DNA epigenetic reprogramming, it was shown that the acetylation/deacetylation of histones mediated by the enzymes HAT-1 (histone acetyltransferase 1) and HDAC-8 (histone deacetylase 8) predominates, especially in individual PAO1 infections, indicating a generalized transcriptional level decrease. Contrary, DNA methylation mediated by DNMT-5 (DNA methyltransferase) generally showed repression of its expression in all infection conditions, especially at the cuticle level, indicating DNA methylation at lower levels that would favor transcription processes **(****Figure 6****).**

### Citation List

Ménard G, Rouillon A, Cattoir V, Donnio PY. Galleria mellonella as a suitable model of bacterial infection: past, present and future. Front Cell Infect Microbiol 2021;11:1299. https://doi.org/10.3389/FCIMB.2021.782733 .
Campo-Pérez V, Cendra M del M, Julián E, Torrents E. Easily applicable modifications to electroporation conditions improve the transformation efficiency rates for rough morphotypes of fast-growing mycobacteria. N Biotechnol 2021;63:10-8. https://doi.org/10.1016/J.NBT.2021.02.003.
Carroll P, Schreuder LJ, Muwanguzi-Karugaba J, Wiles S, Robertson BD, Ripoll J, et al. Sensitive detection of gene expression in mycobacteria under replicating and non-replicating conditions using optimized far-red reporters. PLoS One 2010;5. https://doi.org/10.1371/JOURNAL.PONE.0009823.
Brambilla C, Llorens-Fons M, Julián E, Noguera-Ortega E, Tomàs-Martínez C, Pérez-Trujillo M, et al. Mycobacteria clumping increase their capacity to damage macrophages. Front Microbiol 2016;7:1562. https://doi.org/10.3389/FMICB.2016.01562
Moya-Andérico L, Admella J, Fernandes R, Torrents E. Monitoring gene expression during a Galleria mellonella bacterial infection. Microorganisms 2020;8:1-14. https://doi.org/10.3390/MICROORGANISMS8111798.
Klausen M, Heydorn A, Ragas P, Lambertsen L, Aaes-Jørgensen A, Molin S, et al. Biofilm formation by Pseudomonas aeruginosa wild type, flagella and type IV pili mutants. Mol Microbiol 2003;48:1511-24. https://doi.org/10.1046/J.1365-2958.2003.03525.X.
Admella J, Torrents E. A straightforward method for the isolation and cultivation of Galleria mellonella hemocytes. Int J Mol Sci 2022;23:13483. https://doi.org/10.3390/IJMS232113483.

## Claims

1. *Mycobacterium brumae,* a cell lysate, a cell-free extract, a component, or a culture supernatant thereof, for use in the prevention and/or treatment of an infection or of a disease caused by an infection.

2. *Mycobacterium brumae* for use in the prevention and/or treatment of an infection or of a disease caused by an infection, wherein the *Mycobacterium brumae* is viable.

3. The *Mycobacterium brumae,* cell lysate, cell-free extract, component, or culture supernatant thereof, for use according to any one of claims 1-2, wherein the *Mycobacterium brumae,* cell lysate, cell-free extract, component, or a culture supernatant thereof, is the sole active ingredient.

4. The *Mycobacterium brumae,* cell lysate, cell-free extract, component, or culture supernatant thereof, for use according to any one of claims 1-3, wherein the *Mycobacterium brumae,* cell lysate, cell-free extract, component, or culture supernatant thereof, is administered in a preventively or therapeutically effective amount.

5. The *Mycobacterium brumae,* cell lysate, cell-free extract, component, or culture supernatant thereof, for use according to any one of claims 1-4, wherein the infection is caused by a bacterium, in particular an antibiotic-resistant bacterium.

6. The *Mycobacterium brumae,* cell lysate, cell-free extract, component, or culture supernatant thereof, for use according to any one of claims 1-5, wherein the infection is caused by a pathogen, in particular a bacterium selected from the group consisting of *Pseudomonas aeruginosa, Klebsiella pneumoniae, Salmonella, Vibrio, Mycobacterium, Escherichia coli, Streptococcus, Staphylococcus, Haemophilus, Enterococcus, Clostridium, Neisseria, Helicobacter, Listeria, Chlamydia* and *Acinetobacter baumannii,* and combinations thereof.

7. The *Mycobacterium brumae,* cell lysate, cell-free extract, component, or culture supernatant thereof, for use according to any one of claims 1-6, wherein the use comprises administering a unit dose comprising from 10⁶ to 10¹² cells of *Mycobacterium brumae,* in particular from 10⁹ to 10¹² cells of *Mycobacterium brumae.*

8. The *Mycobacterium brumae,* cell lysate, cell-free extract, component, or culture supernatant thereof, for use according to any one of claims 1-7, wherein the use is preventive.

9. A composition comprising a preventively or therapeutically effective amount of *Mycobacterium brumae,* a cell lysate, a cell-free extract, a component, or a culture supernatant thereof, together with acceptable excipients and/or carriers for use in the prevention and/or treatment of an infection or of a disease caused by an infection.

10. A composition comprising a preventively or therapeutically effective amount of *Mycobacterium brumae,* together with acceptable excipients and/or carriers for use in the prevention and/or treatment of an infection or of a disease caused by an infection, wherein the *Mycobacterium brumae* is viable.

11. The composition for use according to any one of claims 9-10, wherein the *Mycobacterium brumae,* cell lysate, cell-free extract, component, or culture supernatant thereof, is the sole active ingredient.

12. The composition for use according to any one of claims 9-11, wherein the infection is caused by a bacteria, in particular, a bacterium selected from the group consisting of *Pseudomonas aeruginosa, Klebsiella pneumoniae, Salmonella, Vibrio, Mycobacterium, Escherichia coli, Streptococcus, Staphylococcus, Haemophilus, Enterococcus, Clostridium, Neisseria, Helicobacter, Listeria, Chlamydia* and *Acinetobacter baumannii,* and combinations thereof.

13. The composition for use according to any one of claims 9-12, wherein the composition is a pharmaceutical composition and further comprises pharmaceutically acceptable excipients and/or carriers.

14. The composition for use according to any one of claims 9-13, wherein the preventively or therapeutically effective amount of *Mycobacterium brumae* is from 10⁶ to 10¹² cells, in particular from 10⁹ to 10¹² cells.

15. The composition for use according to any one of claims 9-14, wherein the use is preventive.
